# EUROPEAN PATENT APPLICATION

(11) **EP 3 101 618 A1**
(43) Date of publication of application: **07.12.2016**
(21) Application number: 15809492.0
(22) Date of filing: 19.06.2015
(51) Int. Cl.: G06Q 50/24, G06Q 50/22

(54) **DRUG PRESCRIPTION ASSISTANCE METHOD, COMPUTER PROGRAM FOR DRUG PRESCRIPTION ASSISTANCE, AND DRUG PRESCRIPTION ASSISTANCE DEVICE**

(30) Priority: 20.06.2014 JP 2014127334
(71) Applicant: Panasonic Healthcare Holdings Co., Ltd., Tokyo 105-8433 (JP)
(72) Inventor: MINEMURA Atsushi, Ehime 791-0395 (JP); OZAKI Nobuhiko, Ehime 791-0395 (JP); SUZUKI Tetsu, Ehime 791-0395 (JP)
(74) Representative: Grünecker Patent- und Rechtsanwälte PartG mbB
(86) International application number: PCT/JP2015/067805
(87) International publication number: WO 2015/194674

(57) **Abstract**

A drug prescription assistance method in which a server (2) supplies information about a drug to a user terminal (1) via a communication network (N), wherein the method comprises: a step in which the server (2) receives patient data and prescription drug data from the user terminal (1); a step for determining, on the basis of the patient data and data on subscribers to a designated medical plan, whether the patient corresponding to the patient data is a subscriber to the designated medical plan; and a step in which, when the patient is a subscriber to the designated medical plan, the matter of whether there is a drug among the prescription drug data for which a generic drug can be substituted is determined on the basis of a database in which original drugs and the corresponding generic drugs are associated with each other, and, when the substitutable generic drug exists, data on substitutable generic drug data is transmitted to the user terminal (1).

## Description

### TECHNICAL FIELD

The present invention relates to a medicine prescription support method for providing information of medicines to a user terminal through a communication network, a medicine prescription supporting computer program for causing a computer to execute steps of the medicine prescription support method to provide information of medicines to a user terminal through a communication network, and a medicine prescription support apparatus for providing information of medicines to a user terminal through a communication network.

### BACKGROUND ART

A patient who has received a prescription prescribed by a medical institution such as a hospital submits the prescription to a pharmacy. A pharmacist dispenses medicines based on the prescription submitted by the patient and sells the medicines. In recent years, due to the spread of portable terminals, there has been proposed a technique in which a patient can use his/her portable terminal to reserve a pharmacy based on a prescription prescribed by a medical institution such as a hospital and to submit information of the prescription to the pharmacy. That is, the patient reserves a pharmacy using his/her portable terminal, and further transmits prescription information to a pharmacy terminal of the reserved pharmacy through a communication network (for example, see Patent Document 1).

Medicines include original medicines developed and sold earlier and generic medicines containing equivalent components to those of the original medicines. The generic medicines are generally available at a lower price. When there is a generic medicine that can replace an original medicine written in a prescription, the original medicine may be replaced by the generic medicine for dispensing. When the original medicine is replaced by the generic medicine, the burden of medical expense on a patient can be reduced. Reduction in medical expense is desirable for a medical insurer as well as the patient.

### PRIOR ART DOCUMENT

### PATENT DOCUMENT

Patent Document 1: JP-A-2007-148608

### SUMMARY OF THE INVENTION

### PROBLEMS TO BE SOLVED BY THE INVENTION

Every medical insurer has a different understanding on medicines. Some medical insurers want to recommend insured persons to select generic medicines to reduce the burden of medical expenses. However, it is not easy to urge each insured person to spontaneously select generic medicines. For medical insurers who want each insured person to select generic medicines, it is desired to make it easy in a simple manner for the insured person to select generic medicines.

The present disclosed technique proposes a technique by which, of patients who use their user terminals to request preparation of medicines to pharmacies through a communication network, insured persons of a medical insurer wanting the insured persons to select generic medicines can select the generic medicines easily in a simple manner.

### MEANS FOR SOLVING THE PROBLEM

A medicine prescription support method achieved by an aspect of the present disclosed technique is a medicine prescription support method in a server which provides information of medicines to a user terminal through a communication network, the medicine prescription support method, executed by the server, including the steps of: receiving patient data and prescription medicine data from the user terminal; determining whether a patient corresponding to the patient data is a subscriber to a predetermined medical insurance based on subscriber data to the predetermined medical insurance and the patient data; and when the patient is a subscriber to the predetermined medical insurance, determining whether there is a medicine that can be replaced by a generic medicine in the prescription medicine data based on a database in which original medicines are associated with generic medicines corresponding thereto, and when there is a medicine that can be replaced by a generic medicine, transmitting data of the replaceable generic medicine to the user terminal.

A medicine prescription supporting computer program achieved by an aspect of the present disclosed technique is a medicine prescription supporting computer program which causes a computer to execute each step of the above-described medicine prescription support method, so as to provide information of medicines to a user terminal.

A medicine prescription support apparatus achieved by an aspect of the present disclosed technique is a medicine prescription support apparatus which provides information of medicines to a user terminal through a communication network, the medicine prescription support apparatus including: a reception unit which receives patient data and prescription medicine data from the user terminal; a first determination unit which determines whether a patient corresponding to the patient data is a subscriber to a predetermined medical insurance based on subscriber data to the predetermined medical insurance and the patient data; a second determination unit which, when the patient is a subscriber to the predetermined medical insurance, determines whether there is a medicine that can be replaced by a generic medicine in the prescription medicine data based on a database in which original medicines are associated with generic medicines corresponding thereto; and a transmission unit which, when there is a medicine that can be replaced by a generic medicine, transmits data of the replaceable generic medicine to the user terminal.

### ADVANTAGE OF THE INVENTION

According to a medicine prescription support method, a medicine prescription supporting computer program and a medicine prescription support apparatus according to the present disclosed technique, insured persons of a medical insurer wanting the insured persons to select generic medicines can select the generic medicines easily in a simple manner.

### BRIEF DESCRIPTION OF THE DRAWINGS

Fig. 1 is a configuration diagram showing an example of a medicine prescription support system according to the present disclosure.
Fig. 2 is a table showing an example of a database stored in a specified pharmacy information storage unit within a user terminal according to the present disclosure.
Fig. 3 is a table showing an example of a database stored in a prescription information storage unit according to the present disclosure.
Fig. 4 is a table showing the example of the database stored in the prescription information storage unit according to the present disclosure, the view showing a part following Fig. 3.
Fig. 5 is a table showing an example of a database stored in a medical insurance subscriber identification information storage unit within a server according to the present disclosure.
Fig. 6 is a table showing an example of a database stored in an original medicine to generic medicine change correspondence information storage unit within the server according to the present disclosure.
Fig. 7 is a table showing an example of a database stored in a medicine price information storage unit within the server according to the present disclosure.
Fig. 8 is a table showing an example of a database stored in a pharmacy stock information storage unit within the server according to the present disclosure.
Fig. 9 is a chart showing an example of a processing flow of the medicine prescription support system according to the present disclosure.
Fig. 10 is a view showing an example of a screen on which two-dimensional codes can be read in a user terminal according to the present disclosure.
Fig. 11 is a view showing an example of a screen on which prescription information can be checked in the user terminal according to the present disclosure.

### MODE FOR CARRYING OUT THE INVENTION

An embodiment showing an example of a medicine prescription support method, a medicine prescription supporting computer program and a medicine prescription support apparatus according to the present disclosure will be described below in detail with reference to the drawings.

### (Embodiment 1)

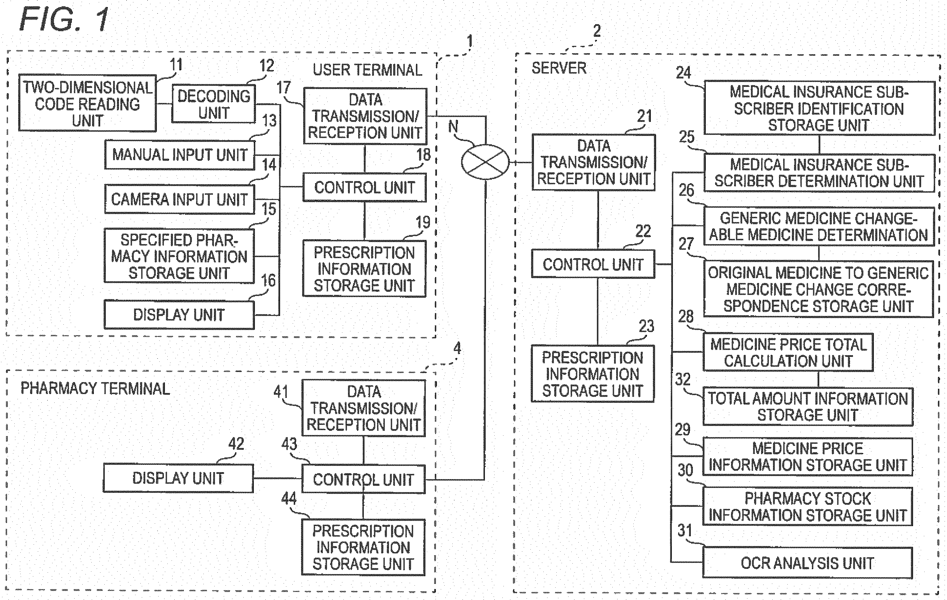

Fig. 1 is a configuration diagram showing an example of a medicine prescription support system according to the present disclosure. The medicine prescription support system is constituted by a user terminal I, a server 2 and a pharmacy terminal 4. The user terminal 1 and the pharmacy terminal 4 are connected to the server 2 through a communication network N individually. The user terminal 1 is a terminal that is operated by a user who has received a prescription prescribed by a medical institution such as a hospital. The user terminal 1 may be, for example, a portable terminal that can be carried by the user. The user terminal 1 may be, for example, used by a patient who has used a hospital or used by a protector of the patient. The pharmacy terminal 4 is a terminal placed in a pharmacy.

The user terminal 1 has a two-dimensional code reading unit 11, a decoding unit 12, a manual input unit 13, a camera input unit 14, a specified pharmacy information storage unit 15, a display unit 16, a data transmission/reception unit 17, a control unit 18, and a prescription information storage unit 19.

The two-dimensional reading unit 11 reads two-dimensional codes written in a prescription. The decoding unit 12 decodes the two-dimensional codes and converts the two-dimensional codes into electronic information. The manual input unit 13 is an input unit through which prescription information can be input manually. The manual input unit 13 is, for example, an operation button, a touch panel, etc.

The camera input unit 14 takes a picture of a prescription in which no two-dimensional code has been written. Incidentally, the two-dimensional code reading unit 11, the manual input unit 13 or the camera input unit 14 is a unit for acquiring data written in a prescription. At least one of the two-dimensional code reading unit 11, the manual input unit 13 or the camera input unit 14 may be provided.

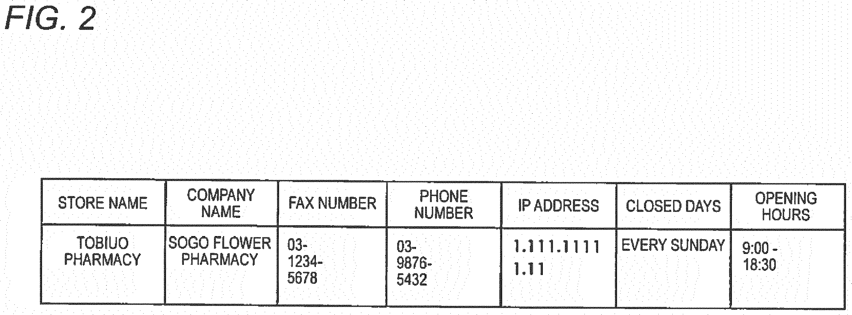

The specified pharmacy information storage unit 15 stores information about a pharmacy that can be specified by a user. Information of the specified pharmacy is input from the two-dimensional code reading unit 11, the manual input unit 13, the camera input unit 14 or the data transmission/reception unit 17. The information of the specified pharmacy may be received as data through the communication network N from a database stored on the Internet or the server 2. Fig. 2 is a table showing an example of a database stored in the specified pharmacy information storage unit 15. In the specified pharmacy information storage unit 15, a store name, a company name, a FAX number, a phone number, an IP address, closed days, and opening hours are stored. However, the data are not limited to those items.

The display unit 16 displays information of prescription read out or a result of data transmitted/received to/from the server 2. The data transmission/reception unit 17 transmits/receives data to/from the server 2 and the pharmacy terminal 4. The control unit 18 controls those processings.

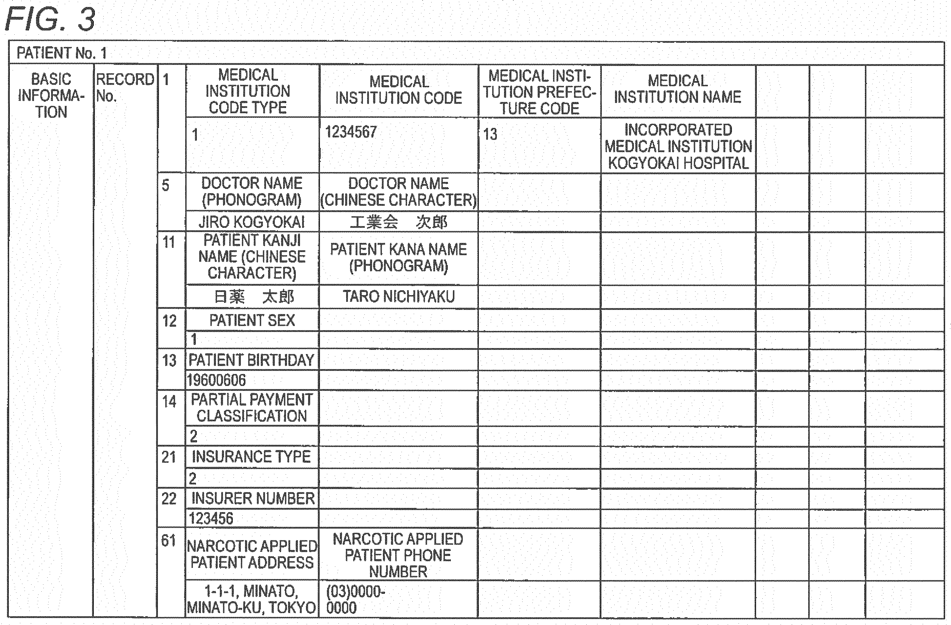

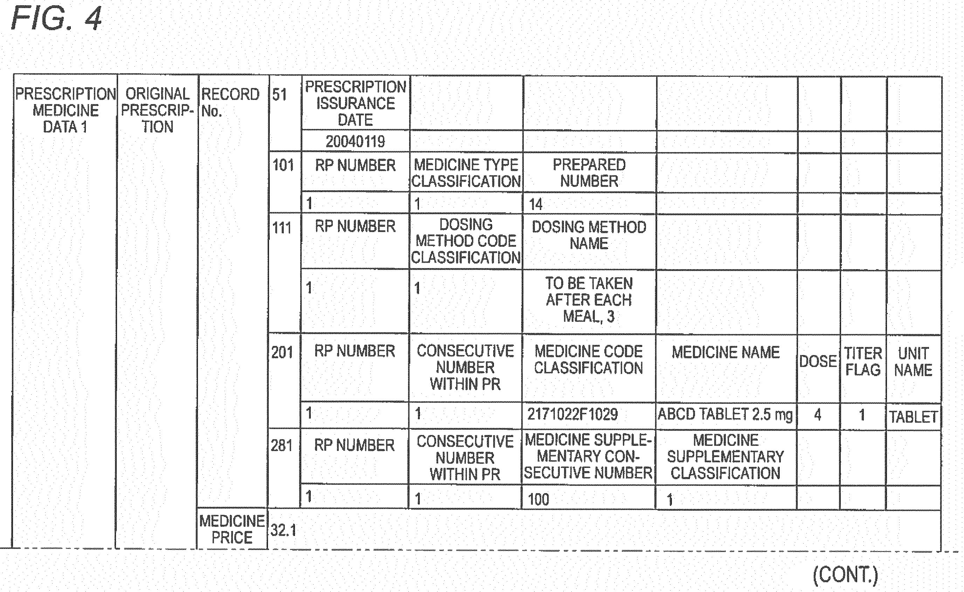

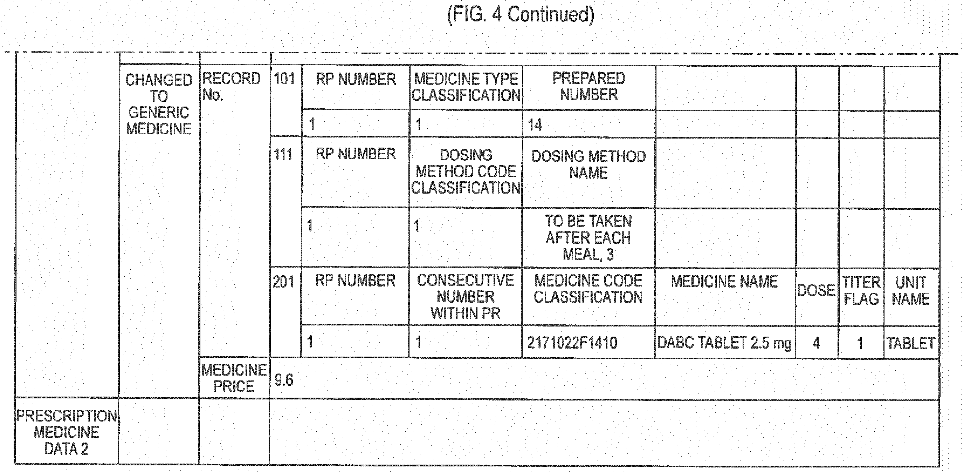

The prescription information storage unit 19 stores prescription information read out or prescription information resulting from transmission/reception to/from the server 2. Fig. 3 and Fig. 4 are tables showing an example of a database stored in the prescription information storage unit 19. The database includes basic data (patient data) and prescription medicine data. Fig. 3 and Fig. 4 show a database created based on a prescription issued once for a patient. When a prescription is issued once, basic data in Fig. 3 and prescription medicine data in Fig. 4 are created individually. The prescription medicine data in Fig. 4 are information following the basic data in Fig. 3. The data in Fig. 3 and the data in Fig. 4 are stored together as a single database. In addition, this database is an example of a database stored when prescription information is read out by two-dimensional codes printed in a prescription. The two-dimensional codes printed in the prescription include basic data and prescription medicine data. The basic data and the prescription medicine data belonging to the two-dimensional codes are stored in the database. Each record No. (number) designates a major category of an item. For example, the record No. 1 designates a medical institution record; No. 5, a doctor record; No. 11, a patient name record; No. 12, a patient sex record; No. 13, a patient birthday record; No. 14, a patient partial payment classification record; No. 21, an insurance type record; No. 22, an insurer number record; and No. 61, a narcotic application record. Categories in the medical institution record include minor category items such as a medical institution code type, a medical institution code, a medical institution prefecture code, a medical institution name, etc. For example, a predetermined number such as 1 designating a medical department, 3 designating a dental department, and 6 designating a visit is recorded in the medical institution code type. The medical institution code is a code with which a receipt should be submitted. The medical institution prefecture code is a number assigned to each prefecture. Chinese characters (kanji) of a medical institution name are recorded in the medical institution name. Items of the basic data are not limited to those items. The prescription medicine data are stored for every individual medicine written in a prescription. The prescription medicine data are classified into an item of original prescription and an item of changed to generic medicine. The original prescription is based on contents described in the two-dimensional codes printed in the prescription issued by the medical institution. For example, each of the medicine is classified into the item of record number and the item of medicine price. The record No. 51 is a prescription issuance date record; No. 101, a medicine type record; No. 111, a dosing method record; No. 201, a medicine record; and No. 281, a medicine supplementary record. Each record further includes minor category items. In the medicine price item, the price of corresponding medicine is recorded. In the item of changed to generic medicine, when the medicine is changed to a generic medicine based on the original prescription, information on the changed generic medicine is recorded. The items of the prescription medicine data are not limited thereto. A part of data may be acquired manually or by reading with an OCR. For example, the insurer number and symbol may be recorded in the basic data field of the prescription information storage unit 19 by receiving information by manual input from the user terminal 1 in advance.

Incidentally, "ABCD tablet" and "DABC tablet" are written in the medicine record No. 201. In fact, a specific tradename of a medicine is written in the alphabetical part of "ABCD" and "DABC". The tradename may include a registered tradename. A similar part in another drawing may be interpreted in the same manner.

The server 2 has a data transmission/reception unit 21, a control unit 22, a prescription information storage unit 23, a medical insurance subscriber identification information storage unit 24, a medical insurance subscriber determination unit 25, a generic medicine changeable medicine determination unit 26, an original medicine to generic medicine change correspondence information storage unit 27, a medicine price total calculation unit 28, a total amount information storage unit 32, a medicine price information storage unit 29, a pharmacy stock information storage unit 30, and an OCR analysis unit 31. The server 2 functions as a medicine prescription support apparatus that can provide information of medicines to the user terminal 1 through the communication network N.

The data transmission/reception unit 21 transmits/receives data to/from the user terminal 1 or the pharmacy terminal 4. The control unit 22 controls processings entirely. The prescription information storage unit 23 accumulates prescription information sent from the user terminal 1. A database recorded in the prescription information storage unit 23 is similar to that in Fig. 3 and Fig. 4.

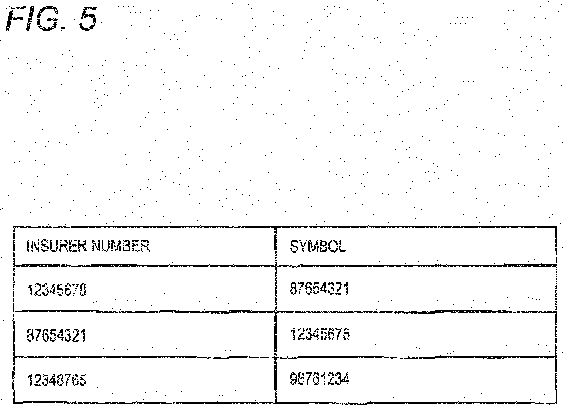

The medical insurance subscriber identification information storage unit 24 accumulates information of predetermined medical insurance subscribers. Insurer numbers of subscribers to medical insurance wanting to make a proposal to users as to change to generic medicines are stored in the medical insurance subscriber identification information storage unit 24. When the medical insurance is, for example, health insurance in Japan, insurer numbers and symbols of health insurance association members are stored in the medical insurance subscriber identification information storage unit 24. In this case, it will go well if at least the insurer numbers are stored in the medical insurance subscriber identification information storage unit 24. Fig. 5 is a table showing an example of a database stored in the medical insurance subscriber identification information storage unit 24. The database in the medical insurance subscriber identification information storage unit 24 may be changed and overwritten.

The medical insurance subscriber determination unit (first determination unit) 25 is connected to the medical insurance subscriber identification information storage unit 24, so as to compare an insurer number etc. included in medical insurance patient information sent from a user terminal with insurer numbers stored in the medical insurance subscriber identification information storage unit 24, and determine whether the patient is a subscriber to predetermined insurance or not.

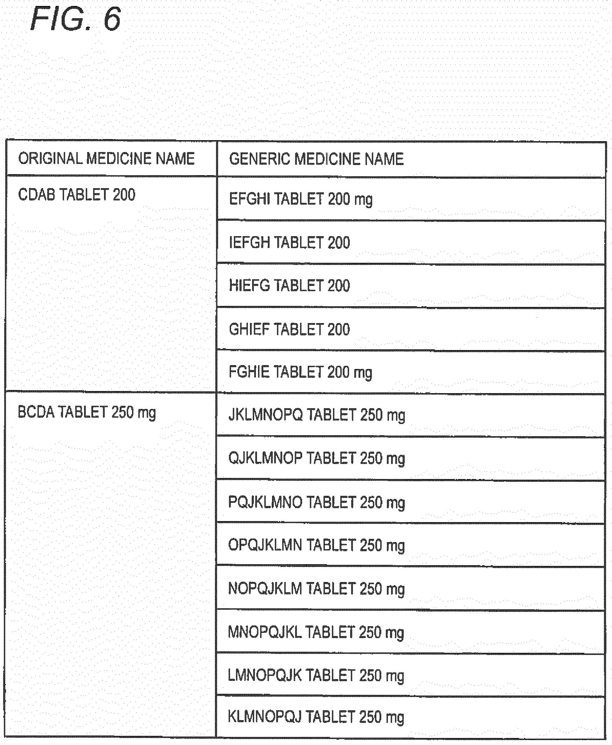

The original medicine to generic medicine change correspondence information storage unit 27 accumulates information about generic medicines corresponding to original medicines so that each original medicine can be paired with a corresponding generic medicine. Fig. 6 is a table showing an example of a database stored in the original medicine to generic medicine change correspondence information storage unit 27. For example, a generic medicine such as an "EFGHI tablet" is present for a "CDAB tablet" that is one of original medicines. Here, each original medicine is stored to be paired with a generic medicine that can replace the original medicine. However, each general name defined by a name of a major component may be stored to be paired with a generic medicine that can replace the general name. In addition, each original medicine may be associated with a plurality of generic medicines that can replace the original medicine.

The generic medicine changeable medicine determination unit (second determination unit) 26 is connected to the original medicine to generic medicine change correspondence information storage unit 27, so as to check whether there is a generic medicine for each medicine within prescription information, and determine whether the original medicine can be replaced by the generic medicine. Incidentally, when medicine information written in a prescription includes a general name defined by the name of a major component, it is determined whether there is or not a generic medicine that can replace a medicine specified by the general name.

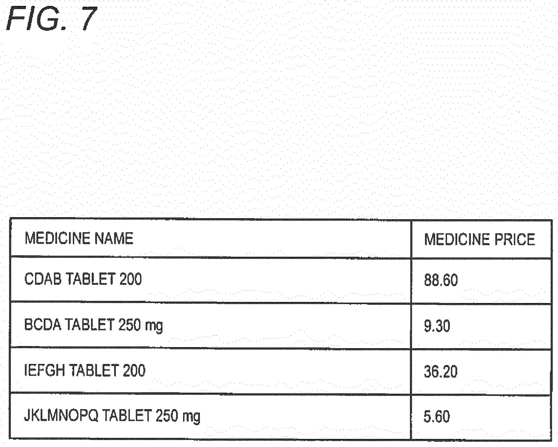

The medicine price information storage unit 29 stores information about medicine prices of original medicines and generic medicines. Fig. 7 is a table showing an example of a database stored in the medicine price information storage unit 29. The medicine price total calculation unit 28 calculates the total amount of medicine prices of prepared medicines including the case where an original medicine has been replaced by a generic medicine.

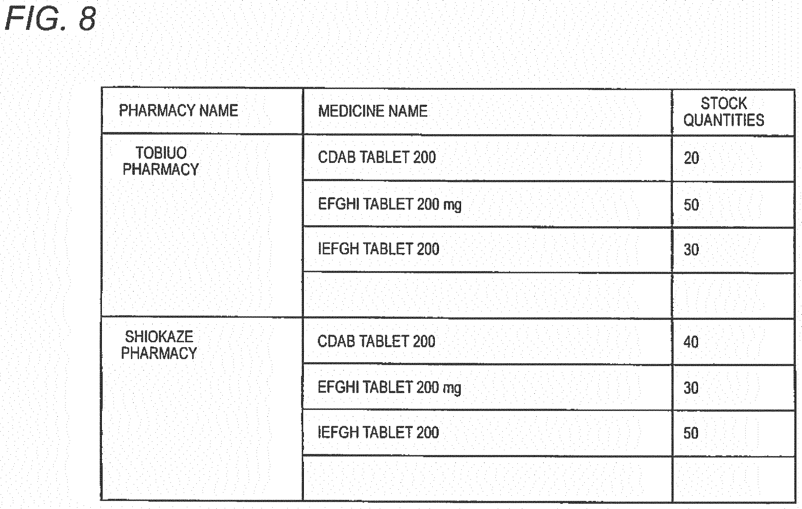

The total amount information storage unit 32 stores the total amount of medicine prices calculated by the medicine price calculation unit 28. The pharmacy stock information storage unit 30 stores information about medicine stock of a specified pharmacy specified by a user. Fig. 8 is a table showing an example of a database stored in the pharmacy stock information storage unit 30. Stock quantities of respective medicines for each pharmacy are stored in the pharmacy stock information storage unit 30. The information about the stock quantities is received from each pharmacy terminal through the communication network N, and recorded in the database. The stock quantities may be input manually through each pharmacy terminal 4. Alternatively, after quantities are input when medicines are delivered, reduction in stock quantities may be recorded and transmitted to the server 2 whenever sales processing is performed in an apparatus (not shown) for performing sales processing in each pharmacy.

The OCR analysis unit 31 analyzes a camera photographed image of a prescription transmitted from the user terminal 1 and converts the image into electronic information. The OCR analysis unit 31 may be removed when the user terminal 1 is not provided with the camera input unit 14.

The pharmacy terminal 4 has a data transmission/reception unit 41, a control unit 43, a prescription information storage unit 44, and a display unit 42. The data transmission/reception unit 41 transmits/receives data to/from the user terminal 1. The control unit 43 controls processings entirely.

The prescription information storage unit 44 accumulates prescription information sent from the user terminal 1. A database recorded in the prescription information storage unit 44 is similar to that in Fig. 3 and Fig. 4. The display unit 42 displays received prescription information.

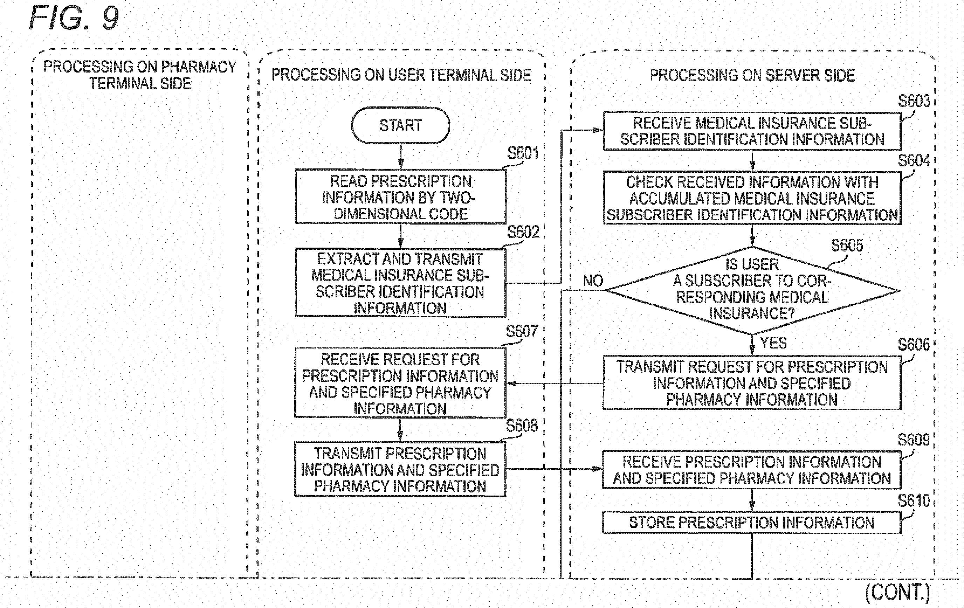

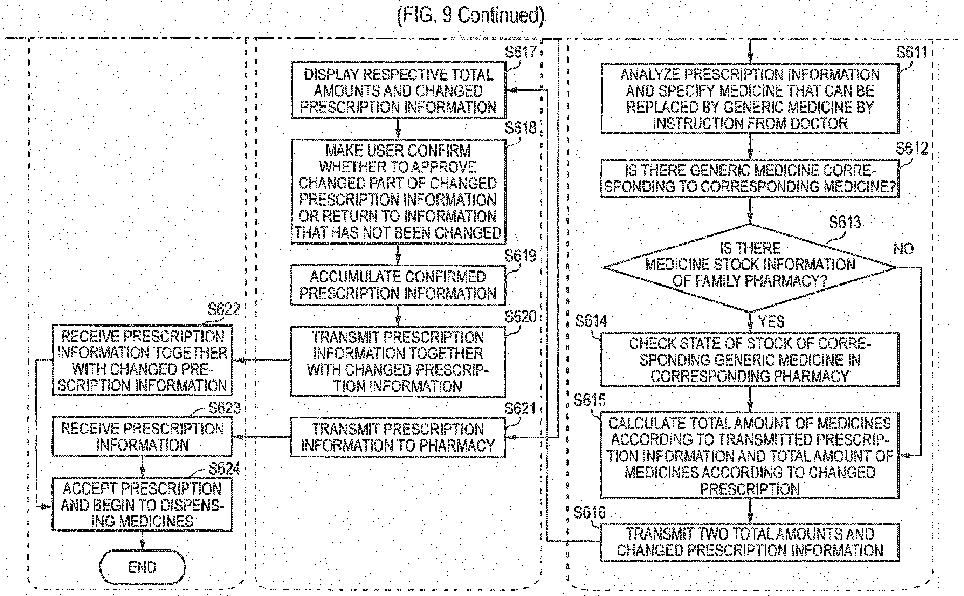

A processing flow to be executed by each configuration will be described below. Fig. 9 is a chart showing an example of a processing flow in the medicine prescription support system disclosed herein. Here, an example will be shown about the case where prescription information is read by the two-dimensional code reading unit 11 of the user terminal 1.

First, the two-dimensional code reading unit 11 reads out prescription information from two-dimensional codes. The decoding unit 12 decodes the read data and converts the data into electronic information. The electronic information is accumulated in the prescription information storage unit 19 (Step S601). The control unit 18 extracts an insurer number as medical insurance subscriber identification information from the database stored in the prescription information storage unit 19. The insurer number is transmitted to the server 2 through the data transmission/reception unit 17 (Step S602). Incidentally, an insurer symbol may be transmitted together.

The data transmission/reception unit 21 of the server 2 receives the insurer number (Step S603).

Under the control of the control unit 22, the medical insurance subscriber determination unit 25 reads out medical insurance subscriber identification information accumulated in the medical insurance subscriber identification information storage unit 24, and compares the medical insurance subscriber identification information with the received medical insurance subscriber identification information (insurer number) (Step S604). Thus, the medical insurance subscriber determination unit 25 determines whether the user is a subscriber to a predetermined medical insurance or not (Step S605).

When the user is not a subscriber to the medical insurance (No in Step S605), the control unit 22 transmits the comparison result to the data transmission/reception unit 17 of the user terminal 1 through the data transmission/reception unit 21. Then, a screen for confirming whether to transmit prescription information to a specified pharmacy is displayed on the display unit 16. Using the manual input unit 13, the user gives an instruction to transmit prescription information to the pharmacy. Prescription information accumulated in the prescription information storage unit 19 is transmitted to an IP address of a pharmacy recorded in the specified pharmacy information storage unit 15, that is, to the data transmission/reception unit 41 of the pharmacy terminal 4 through the data transmission/reception unit 17 (Step S621). Incidentally, when the user is not a medical insurance subscriber (No in Step 605), the control unit 22 may transmit the prescription information from the server 2 to the pharmacy terminal 4 of the specified pharmacy without interposing the user terminal 1.

Under the control of the control unit 43, the data transmission/reception unit 41 receives the prescription information and accumulates the prescription information in the prescription information storage unit 44 (Step S623). Under the control of the control unit 43, the display unit 42 displays the prescription information. A pharmacist accepts the prescription and begins to dispense medicines (Step S624)

On the other hand, when the user is a medical insurance subscriber (Yes in Step S605), the control unit 22 of the server 2 uses the data transmission/reception unit 21 to transmit a signal to the data transmission/reception unit 17 of the user terminal I (Step S606). The signal is a request to transmit prescription information and specified pharmacy information from the user terminal 1 to the server 2. The data transmission/reception unit 17 receives the request signal for prescription information and specified pharmacy information (Step S607). The control unit 18 reads out prescription information accumulated in the prescription information storage unit 19 and specified pharmacy information accumulated in the specified pharmacy information storage unit 15, and transmits the prescription information and the specified pharmacy information to the data transmission/reception unit 21 of the server 2 through the data transmission/reception unit 17 (Step S608). The data transmission/reception unit 21 receives the two kinds of information (Step S609).

The control unit 22 stores the prescription information in the prescription information storage unit 23 (Step S610). Then, medicine information within the information database is checked as to doctor's propriety of change to generic medicines, and medicines that can be changed to generic medicines are specified (Step S611). For example, of the prescription information storage unit 23 described in Fig. 3 and Fig. 4, a medicine for which a predetermined numerical value, for example, 3 is placed in the medicine supplementary classification written in the record No. 281 is determined as not permitted to be changed to a generic medicine.

Under the control of the control unit 22, the generic medicine changeable medicine determination unit 26 reads out original medicine to generic medicine change correspondence information accumulated in the original medicine to generic medicine change correspondence information storage unit 27 for each of the medicines that can be changed to generic medicines, and extracts corresponding generic medicines. Further, respective medicine prices of the extracted generic medicines are read out from the medicine price information storage unit 29, and changeable generic medicines are specified (Step S612). Incidentally, when there are a plurality of generic medicines, a medicine at a lowest price may be specified.

Next, whether there is or not stock information of each medicine in the pharmacy specified by the specified pharmacy information is checked in the pharmacy stock information storage unit 30 (Step S613).

When the stock information is present (Yes in Step S613), the control unit 22 reads out information of each medicine in the corresponding pharmacy from the pharmacy stock information storage unit 30, and information about, of the generic medicines specified in Step S612, equivalent generic medicines in stock is recorded in the prescription information storage unit of the server (Step S614). When there are a plurality of generic medicines in stock, a medicine at a lowest price may be extracted and recorded.

Further, the medicine price total calculation unit 28 calculates the total amount according the change to the medicines extracted in Step S612 or S614 and the total amount of medicines according to the prescription received in Step S609 (Step S615). The server 2 temporarily stores the calculated total amounts into the total amount information storage unit 32.

The control unit 22 transmits the aforementioned two total amounts and the prescription information changed in Step S612 or S614 to the data transmission/reception unit 17 of the user terminal 1 through the data transmission/reception unit 21 (Step S616). Incidentally, when there are a plurality of replaceable generic medicines, a plurality of candidates may be transmitted to the user terminal 1 so that the user can select one in Step S617 as will be described later.

Incidentally, when there is no medicine stock information for the specified pharmacy in Step S613 (No in Step S613), Step S614 is skipped, and Step S615 is carried out.

When the control unit 18 receives the aforementioned information through the data transmission/reception unit 17, the control unit 18 uses the display unit 16 to display the information for the user (Step S617). Here, the manual input unit 13 accepts confirmation from the user as to whether to approve the changed part of the changed prescription information or return to the information that has not been changed (Step S618). The control unit 18 accumulates the approved changed prescription information in the prescription information storage unit 19 (Step S619). Further, with reference to the specified pharmacy information of the specified pharmacy information storage unit 15, the control unit 18 transmits the approved changed prescription information to the data transmission/reception unit 41 of the pharmacy terminal 4 through the data transmission/reception unit 17 (Step S620).

In the pharmacy terminal 4, the data transmission/reception unit 41 receives the prescription information (Step S622). The control unit 43 accumulates the information in the prescription information storage unit 44 and displays the information on the display unit 42. A pharmacist accepts the prescription and begins to dispense medicines (Step S624).

Here, when not only data according to the change to generic medicines but also data according to the original prescription are received and displayed, the pharmacist can also confirm medicines written in the prescription issued from the medical institution on the pharmacy terminal. Thus, the pharmacy can examine the propriety of the change to the generic medicines. Safety can be further improved.

Incidentally, here, when the two-dimensional codes are absent from the prescription, a prescription image photographed by the camera input unit 14 of the user terminal 1 may be transmitted to the server through the data transmission/reception unit 17 in place of Step S602, and the OCR analysis unit 31 may perform OCR analysis on the image and convert the image into electronic information so that medical insurance subscriber information can be extracted from the electronic information.

In addition, in the same manner, when the two-dimensional codes are absent from the prescription, information input manually through the manual input unit 13 may be transmitted to the server 2 through the data transmission/reception unit 17 in place of Step S602.

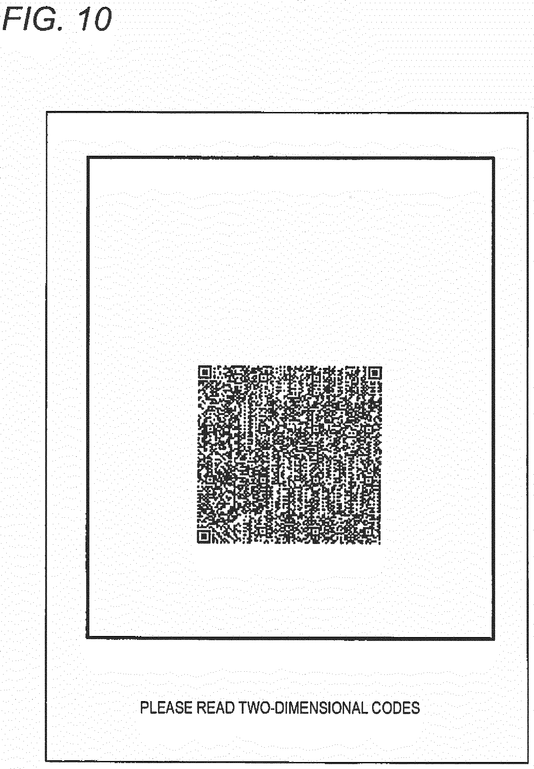

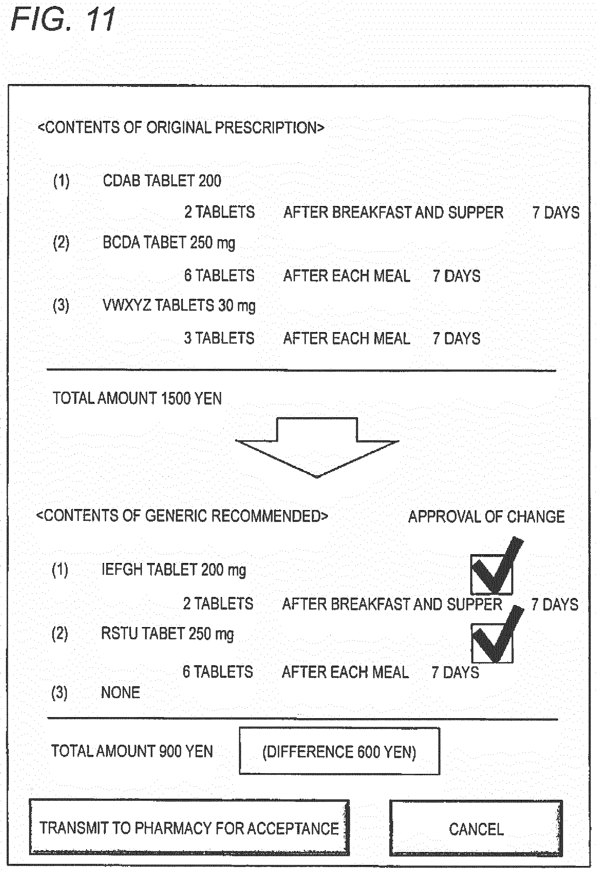

Incidentally, Step S611 may be removed so that Step S612 can follow Step S610. In addition, Steps S613 and S614 may be removed so that Step S615 can follow Step S612. In addition, Step S616 may be also removed. That is, when there are corresponding generic medicines in Step S612, a list of changeable medicines may be transmitted to execute Step S618 and following steps without calculating medicine prices. Fig. 10 is a view showing a screen to be displayed in the user terminal 1 in Step S601. Fig. 10 is a view in which two-dimensional codes are being read. Fig. 11 is a view showing an example of a screen to be displayed in the user terminal 1 in Steps S617 and S618.

In this manner, the medical insurance subscriber identification information storage unit 24 and the medical insurance subscriber determination unit 25 are provided so that, for example, only medical insurance subscribers making an agreement in advance and using this service or this system can be recommended to change original medicines to generic medicines. Each medical insurer may have a different wish as to whether to recommend change to generic medicines or not. According to the technique disclosed herein, change to generic medicines is proposed to, of patients who use their user terminals 1 to request for preparation of medicines to pharmacies through the communication network N, insured persons of a medical insurer wanting the insured persons to select generic medicines, but change to generic medicines is not proposed to insured persons of a medical insurer who does not recommend the insured persons to change to generic medicines. That is, the wish of each insurer can be reflected without making each user be aware of whether the change to generic medicines is proposed or not.

The change of original medicines to generic medicines is an important factor contributing to reduction in medical expense. In any medical insurance in the background art, an amount that can be reduced when original medicines are changed to generic medicines can be merely calculated ex post facto based on receipt data sent three or more months after payment processing in a pharmacy. Thus, recommendation of generic medicines can be made only using the result of the calculation. On the next visit to the pharmacy, the same medicines are not always prepared. In addition, information is presented when three or more months have passed. Therefore, effect of reduction in medical expenses is limited.

When the technique disclosed herein is used, generic medicines can be automatically presented to members of a medical insurer wanting the members to change to the generic medicines before each user who has received a prescription submits the prescription to a pharmacy. Accordingly, the medical insurer wanting the members to change to the generic medicines can expect more effect of reduction in medical expenses than ever. In addition, the amount of medical expense reduced by selection of generic medicines using this service or this system can be made clear for each medical insurer by specifying which medical insurance a user belongs to or whether the medical insurance is a predetermined medical insurance specified in advance. Thus, the value provided by the service or the system can be measured in terms of money.

Incidentally, medical insurance can be roughly classified into public medical insurance and private medical insurance. An insurance system differs from one country to another. For example, it is chiefly expected in Japan to apply this disclosure to the National Health Insurance or health insurance managed by a health insurance society belonging to a company or the like, and it is chiefly expected in the United States to apply this disclosure to medical insurance managed by a private insurance company. That is, this disclosure can be applied to any one of the aforementioned medical insurances.

Incidentally, although a form to be used in a server providing information of medicines to a user terminal through a communication network is chiefly assumed as the medicine prescription support method according to this disclosure, the disclosure is not limited to the form. That is, a medicine prescription support method assumed in this disclosure is a medicine prescription support method executing the steps of: determining whether a patient corresponding to patient data having been input is a subscriber to a predetermined medical insurance or not based on subscriber data to the predetermined medical insurance and the patient data; and when the patient is a subscriber to the predetermined medical insurance, determining whether prescription medicine data having been input includes a medicine that can be replaced by a generic medicine or not based on a database in which original medicines are associated with generic medicines corresponding thereto, and when there is a medicine that can be replaced by a generic medicine, outputting data of the replaceable generic medicine.

By the medicine prescription support method configured thus, insured members of a medical insurer wanting the members to select generic medicines can select the generic medicines easily in a simple manner.

Incidentally, this disclosure is not limited to the aforementioned embodiment, but changes, improvements, etc. can be made suitably. In addition, materials, shapes, dimensions, numbers, forms, arrangement places, etc. of constituent elements in the aforementioned embodiment are not limited but may be selected desirably if the invention can be attained.

The present application is based on a Japanese patent application filed on June 20, 2014, that is, Japanese Patent Application No. 2014-127334, the contents of which are incorporated herein by reference.

### INDUSTRIAL APPLICABILITY

According to the technique disclosed herein, of patients using their user terminals to request for preparation of medicines to pharmacies through a communication network, insured persons of a medical insurer wanting the insured persons to select generic medicines can select the generic medicines easily in a simple manner.

### DESCRIPTION OF REFERENCE NUMERALS AND SIGNS

1: UserTerminal
2: Server (Medicine Prescription Support Apparatus)
4: Pharmacy Terminal
11: Two-Dimensional Code Reading Unit
12: Decoding Unit
13: Manual Input Unit
14: Camera Input Unit
15: Specified Pharmacy Information Storage Unit
16: Display Unit
17: Data Transmission/Reception Unit
18: Control Unit
19: Prescription Information Storage Unit
21: Data Transmission/Reception Unit
22: Control Unit
23: Prescription Information Storage Unit
24: Medical Insurance Subscriber Identification Storage Unit
25: Medical Insurance Subscriber Determination Unit (First Determination Unit)
26: Generic Medicine Changeable Medicine Determination Unit (Second Determination Unit)
27: Original Medicine to Generic Medicine Change Correspondence Storage Unit
28: Medicine Price Total Calculation Unit
29: Medicine Price Information Storage Unit
30: Pharmacy Stock Information Storage Unit
31: OCR Analysis Unit
32: Total Amount Information Storage Unit
41: Data Transmission/Reception Unit
42: Display Unit
43: Control Unit
44: Prescription Information Storage Unit
N: Communication Network

## Claims

1. A medicine prescription support method in a server which provides information of medicines to a user terminal through a communication network, the medicine prescription support method, executed by the server, comprising the steps of:
receiving patient data and prescription medicine data from the user terminal;
determining whether a patient corresponding to the patient data is a subscriber to a predetermined medical insurance based on subscriber data to the predetermined medical insurance and the patient data; and
when the patient is a subscriber to the predetermined medical insurance, determining whether there is a medicine that can be replaced by a generic medicine in the prescription medicine data based on a database in which original medicines are associated with generic medicines corresponding thereto, and when there is a medicine that can be replaced by a generic medicine, transmitting data of the replaceable generic medicine to the user terminal.

2. The medicine prescription support method according to claim 1,
wherein the server makes communication with a pharmacy terminal through a communication network, and
wherein the medicine prescription support method, executed by the server, further comprises the step of:
when the patient is not a subscriber to the predetermined medical insurance, transmitting the prescription medicine data from the server to the pharmacy terminal without determining whether there is a medicine that can be replaced by a generic medicine.

3. The medicine prescription support method according to claim 1,
wherein the server makes communication with a pharmacy terminal through a communication network, and
wherein the medicine prescription support method, executed by the server, further comprises the step of:
when the patient is not a subscriber to the predetermined medical insurance, transmitting, to the user terminal, a signal giving an instruction to transmit the prescription medicine data from the user terminal to the pharmacy terminal without determining whether there is a medicine that can be replaced by a generic medicine.

4. The medicine prescription support method according to any one of claims 1 to 3, wherein the medicine prescription support method, executed by the server, further comprises the step of:
when there are a plurality of generic medicines by which the medicine can be replaced, extracting a generic medicine at a lowest medicine price based on a medicine price database in which amounts of medicines are stored.

5. The medicine prescription support method according to any one of claims 1 to 4, wherein the medicine prescription support method, executed by the server, further comprises the step of:
transmitting an amount of medicines in a case in which the medicine is replaced by the generic medicine and an amount of medicines in a case in which the medicine is not replaced, based on a medicine price database in which amounts of medicines are stored.

6. The medicine prescription support method according to any one of claims 1 to 5, wherein the medicine prescription support method, executed by the server, further comprises the step of:
calculating an amount of medicines in a case in which the medicine is replaced by the generic medicine and an amount of medicines in a case in which the medicine is not replaced, based on a medicine price database in which amounts of medicines are stored; and
transmitting a difference between the two amounts to the user terminal.

7. The medicine prescription support method according to any one of claims 1 to 6,
wherein the prescription medicine data comprise information about propriety of replacement by generic medicines, the propriety being determined by a doctor, and
wherein the medicine prescription support method, executed by the server, further comprises the step of:
when there is a medicine that can be replaced by a generic medicine, checking whether the medicine is determined as replaceable by the generic medicine by the doctor based on the prescription medicine data.

8. The medicine prescription support method according to any one of claims 1 to 7, wherein the medicine prescription support method, executed by the server, further comprises the step of:
determining whether the generic medicine by which the medicine can be replaced is in stock based on a stock database of medicines held by a pharmacy.

9. The medicine prescription support method according to any one of claims 1 to 8,
wherein the patient data comprise an insurer number, and
wherein the subscriber data to the predetermined medical insurance comprise a database in which predetermined insurer numbers are stored.

10. A medicine prescription support method executing the steps of:
determining whether a patient corresponding to patient data having been input is a subscriber to a predetermined medical insurance based on subscriber data to the predetermined medical insurance and the patient data; and
when the patient is a subscriber to the predetermined medical insurance, determining whether there is a medicine that can be replaced by a generic medicine in prescription medicine data having been input based on a database in which original medicines are associated with generic medicines corresponding thereto, and when there is a medicine that can be replaced by a generic medicine, outputting data of the replaceable generic medicine.

11. A medicine prescription supporting computer program which causes a computer to execute each step of the medicine prescription support method according to any one of claims 1 to 10, so as to provide information of medicines to a user terminal.

12. A medicine prescription support apparatus which provides information of medicines to a user terminal through a communication network, the medicine prescription support apparatus comprising:
a reception unit which receives patient data and prescription medicine data from the user terminal;
a first determination unit which determines whether a patient corresponding to the patient data is a subscriber to a predetermined medical insurance based on subscriber data to the predetermined medical insurance and the patient data;
a second determination unit which, when the patient is a subscriber to the predetermined medical insurance, determines whether there is a medicine that can be replaced by a generic medicine in the prescription medicine data based on a database in which original medicines are associated with generic medicines corresponding thereto; and
a transmission unit which, when there is a medicine that can be replaced by a generic medicine, transmits data of the replaceable generic medicine to the user terminal.

13. The medicine prescription support apparatus according to claim 12, wherein when the first determination unit determines that the patient is not a subscriber to the predetermined medical insurance, the second determination unit does not determine whether there is a medicine that can be replaced by a generic medicine, but the transmission unit transmits the prescription medicine data to the pharmacy terminal through the communication network.

14. The medicine prescription support apparatus according to claim 12, wherein when the first determination unit determines that the patient is not a subscriber to the predetermined medical insurance, the second determination unit does not determine whether there is a medicine that can be replaced by a generic medicine, but the transmission unit transmits, to the user terminal, a signal giving an instruction to transmit the prescription medicine data from the user terminal to the pharmacy terminal.
